# EUROPÄISCHE PATENTANMELDUNG

(11) **EP 0 821 950 A2**
(43) Veröffentlichungstag der Anmeldung: **04.02.1998**
(21) Anmeldenummer: 97112339.3
(22) Anmeldetag: 18.07.1997
(51) Int. Cl.: A61K 9/107, A61K 31/015

(54) **Physiologisch wirksame Mengen beta-Carotin-enthaltende klare wässrig-alkoholische Multivitamin-Lösung**

(30) Priorität: 31.07.1996 DE 19630789
(71) Anmelder: BASF AKTIENGESELLSCHAFT, 67056 Ludwigshafen (DE)
(72) Erfinder: Weber, Sabine, 69469 Weinheim (DE); Sambale, Clemens, 67459 Böhl-Iggelheim (DE); End, Lutz, Dr., 68199 Mannheim (DE); Gaus, Günther, Dr., 68647 Biblis (DE)

(57) **Zusammenfassung**

Optisch klare, 40 bis 150 ppm beta-Carotin-enthaltende, wäßrig-alkoholische Multivitaminlösung, erhältlich durch Mischen einer optisch klaren wäßrig-alkoholischen Multivitaminlösung, enthaltend Vitamine in Mengen, die 30% bis 300% des Tagesbedarfs eines Erwachsenen pro Tagesdosis Lösung entsprechen, sowie gegebenenfalls Mineralstoffen in geringen Mengen, mit einer beta-Carotin-Emulsion, die mehr als 2 Gew.-% beta-Carotin, bezogen auf die Emulsion, in der dispergierten Ölphase aus einem eßbaren Öl gelöst, enthält.

## Beschreibung

Die Erfindung betrifft klare wäßrig-alkoholische Multivitamin-Lösungen, die in klarer micellarer Lösung beta-Carotin in physiologisch wirksamen Mengen von z.B. über 40 ppm enthalten und ein Verfahren zur Einbringung des beta-Carotins in die Multivitaminlösungen.

Flüssige Multivitamin-Präparate sind in der Regel trüb und haben oft einen sirupartigen Charakter. Es war jedoch nicht nur wünschenswert, aus optischen Gründen klare Multivitaminlösungen zur Verfügung zu stellen, sondern aus Gründen der guten Bioverfügbarkeit ist es auch zweckmäßig, die Wirkstoffe gelöst anzubieten. Dementsprechend sind bereits vollkommen klare Multivitamin-Lösungen im Handel, z.B. "Centrum Liquid"® der Fa. Lederle (Lederle Consumer Health Division, Pearl River, NY 10965, U.S.A.). Solche Lösungen enthalten im Volumen eines Eßlöffels (15 cc) die erforderliche tägliche Dosis aller gängigen Vitamine sowie gegebenenfalls bestimmte Mineralstoffe.

Beta-Carotin ist aufgrund zahlreicher groß angelegter Untersuchungen ein erwünschter Nahrungszusatzstoff, da es einerseits als Provitamin A fungiert und andererseits eine Wirkung als Radikalfänger im Organismus vermutet wird. Gegenüber dem Zusatz von Vitamin A, das bei Überdosierung toxisch sein kann, hat es zudem den Vorteil, daß eine Überdosierung nicht schadet, da der Organismus nur die erforderlichen Mengen in Vitamin A umwandelt.

Beta-Carotin kommt in der Natur in vielen Pflanzen und Früchten vor. In der Regel wird jedoch mit der heutigen modernen Ernährung nur selten eine ausreichende Zufuhr von beta-Carotin erreicht. Beta-Carotin-Nahrungszusätze sind deshalb regelmäßig mit synthetischem beta-Carotin hergestellt. Dieses synthetisch hergestellte beta-Carotin liegt in der all-trans-Form gut kristallisiert vor. Es ist nur wenig in Ölen und Fetten und etwas besser in Halogenkohlenwasserstoffen löslich. In Alkohol und Wasser ist es bei Raumtemperatur praktisch unlöslich. Halogenkohlenwasserstoffe scheiden verständlicherweise aus toxikologischen Gründen von vornherein aus. Ölige Lösungen konnten bestenfalls zu trüben Emulsionen mit wäßrigen Substraten führen.

Es bestand nun die Aufgabe, beta-Carotin optisch klar gelöst in eine wäßrig-alkoholische Multivitaminlösung einzubringen.

Unter optisch klar wird dabei ein Produkt verstanden, das z.B. eine Extinktion von 0,02-0,08 bei einer Wellenlänge von 700 nm in einer 1-cm-Küvette, d.h. eine Durchlässigkeit von 92-98%, aufweist.

Diese Aufgabe wurde überraschenderweise mit optisch klaren Multivitamin-Lösungen gelöst, die in einer Tagesdosis, z.B. in 15 cc Lösung, übliche Mengen einer täglichen Einzeldosis, z.B. 30-300% des täglichen Bedarfs eines Erwachsenen an Vitaminen in wäßrigalkoholischer Lösung unter Zusatz eines Emulgators enthalten, denen eine solche Menge einer beta-Carotin-Emulsion, die mehr als 2 Gew.-%, vorzugsweise 3-15 Cew.-% beta-Carotin, bezogen auf die Emulsion, enthält und bei der sich das beta-Carotin zusammen mit einem eßbaren Öl in der dispersen Ölphase befindet, zugemischt ist, so daß der beta-Carotin-Gehalt der Multivitamin-Lösung 40 bis 150 ppm beträgt.

Die neuen beta-Carotin-enthaltenden klaren Multivitaminlösungen enthalten entweder nur ausgewählte Vitamine wie Vitamin A, C und E sowie gegebenenfalls Vitamine des Vitamin-B-Komplexes oder enthalten den gesamten Bedarf aus Vitamin A, E, C, B₁, B₂, Niacinamid, B₆, B₁₂, D, Biotin, Pantothensäure sowie ferner gegebenenfalls geringe Mengen von Iod, Eisen, Zink, Mangan, Chrom und Molybdän. Diese klaren wäßrigen Multivitaminlösungen enthalten in der Regel 2 bis 10 Gew.- % Alkohol sowie Emulgatoren vom Typ nichtionische Emulgatoren mit einem HLB-Wert von 12-18. Im einzelnen sind z.B. polyoxethylierte Fettsäuren, polyoxethylierte Triglyceride von Fettsäuren, Polyethylenglykol-Glycerolhydroxystearat (Cremophor® BASF Aktiengesellschaft), polyoxethylierte Ester von Fettsäuren mit Sorbitol (Tween®, Polysorbat), polyoxethyliertes Rhizinusöl (Cremophor), Pluronic zu nennen. Eine repräsentative Multivitaminlösung enthält z.B. die folgenden Vitamine einzeln oder in Kombination

| | |
|---|---|
| Vitamin A | 2500 IU |
| Vitamin E | 30 IU |
| Vitamin C | 60 mg |
| Vitamin B₁ | 1,5 mg |
| Vitamin B₂ | 1,7 mg |
| Niacin | 20 mg |
| Vitamin B₆ | 2 mg |
| Vitamin B₁₂ | 6 mcg |
| Vitamin D2 | 400 IU |
| Biotin | 300 mcg |
| Pantothensäure | 10 mg |
| Iod | 150 mcg |
| Eisen | 9 mg |
| Zink | 3 mg |
| Mangan | 2,5 mg |
| Chrom | 25 mcg |
| MolybdäN | 25 mcg |

Die diesen Multivitaminlösungen zuzumischenden klaren, mehr als 2 Gew.-% beta-Carotin enthaltenden Emulsionen, enthalten in der Regel beta-Carotin in einem eßbaren Öl, z.B. Kokosnußöl, gelöst in der dispersen Ölphase in Konzentrationen von mehr als 2, insbesondere 3 bis 15 Gew.-%, vorzugsweise 5-12 Gew.-%. Geeignete Emulsionen sind z.B. in EP 0551638 beschrieben, wobei als äußere Phase Glycerin oder ein Gemisch aus Glycerin und Wasser sowie Ester der Ascorbinsäure mit langkettigen Fettsäuren als Emulgator und Stabilisator verwendet werden. Deshalb wird auf die Angaben und Erläuterungen dieser Patentschrift ausdrücklich Bezug genommen.

Der Erfolg der Herstellung optisch klarer wäßrig/alkoholischer Multivitaminlösungen mit einem Gehalt von mehr als 40 ppm beta-Carotin war überraschend, da bei Zugabe der beta Carotin-enthaltenden Emulsion das beta-Carotin nicht ausfällt, obwohl es in Wasser und Alkohol praktisch unlöslich ist,

### Beispiele

### Beispiel 1

Für eine Lösung mit 40 ppm beta-Carotin werden 1000 g einer wie oben beschriebenen repräsentative Multivitaminlösung, die als Emulgator Polysorbat 80 enthält, mit 400 mg einer gemäß EP 0551638, Beispiel 4 hergestellten Emulsion mit einem beta-Carotin-Gehalt von 10 Gew.-% bei Raumtemperatur unter mäßigem Rühren gemischt und unter Stickstoffgas in braune Glasflaschen abgefüllt. Die zunächst trübe Lösung ist nach wenigen Tagen klar und bleibt anschließend stabil.

### Beispiel 2

Für eine Lösung mit 100 ppm beta-Carotin werden 1000 g einer wie oben beschriebenen repräsentativen Multivitaminlösung mit 1 g einer gemäß EP 0551638, Beispiel 4 hergestellten Emulsion mit einem beta-Carotin-Gehalt von 10 Gew.-% bei Raumtemperatur unter mäßigem Rühren gemischt und unter Stickstoffgas in braune Glasflaschen abgefüllt. Die zunächst trübe Lösung ist nach 9-11 Tagen klar und bleibt anschließend stabil.

## Patentansprüche

1. Optisch klare, 40 bis 150 ppm beta-Carotin-enthaltende, wäßrig-alkoholische Muitivitaminlösung, erhältlich durch Mischen einer optisch klaren wäßrig-alkoholischen Multivitaminlösung, enthaltend Vitamine in Mengen, die 30% bis 300% des Tagesbedarfs eines Erwachsenen pro Tagesdosis Lösung entsprechen, sowie gegebenenfalls Mineralstoffen in geringen Mengen, mit einer beta-Carotin-Emulsion, die mehr als 2 Gew.-% beta-Carotin, bezogen auf die Emulsion, in der dispergierten Ölphase aus einem eßbaren Öl gelöst, enthält.

2. Optisch klare, beta-Carotin enthaltende Multivitaminlösung gemäß Anspruch 1, erhältlich durch Zumischen einer 3 bis 15 Gew.-% beta-Carotin enthaltenden Emulsion, die in der dispersen Ölphase aus einem eßbaren Öl das beta-Carotin gelöst und in der äußeren Phase Glycerin oder ein Gemisch aus Glycerin/Wasser sowie einen Zusatz von Estern der Ascorbinsäure mit langkettigen Fettsäuren als Emulgator enthält.

3. Optisch klare wäßrig-alkoholische Multivitaminlösung, enthaltend 2 bis 10 Gew.-% Ethanol, 1 bis 30 Gew.-% eines nichtionischen Emulgators mit einem HLB-Wert von 12-18 und Vitamine in Mengen von 30 bis 300% des Tagesbedarfs des Menschen pro Tagesdosis Lösung sowie 40 bis 150 ppm beta-Carotin.
